# EUROPEAN PATENT SPECIFICATION

(11) **EP 2 022 362 B1**
(45) Date of publication and mention of the grant of the patent: **08.11.2017**
(21) Application number: 07743715.0
(22) Date of filing: 18.05.2007
(51) Int. Cl.: A45D 34/04, A61K 8/02, A61K 8/34, A61Q 1/04

(54) **VISCOUS COSMETIC**
VISKOSES KOSMETIKPRODUKT
PRODUIT COSMÉTIQUE VISQUEUX

(30) Priority: 31.05.2006 JP 2006152547
(43) Date of publication of application: 11.02.2009
(73) Proprietor: Mitsubishi Pencil Company, Limited, Tokyo 140-8537 (JP)
(72) Inventor: MIYAZAKI, Takayuki, Yokohama-shi, Kanagawa 224-8558 (JP); FUJIOKA, Tomochika, Yokohama-shi, Kanagawa 224-8558 (JP); KAMITANI, Kazuyuki, Yokohama-shi, Kanagawa 224-8558 (JP); HAMADA, Masafumi, Fujioka-shi, Gunma 375-8501 (JP); KOYAMA, Hiroaki, Fujioka-shi, Gunma 375-8501 (JP); AKAISHI, Tetsuaki, Fujioka-shi, Gunma 375-8501 (JP); FUKUMOTO, Takeo, Fujioka-shi, Gunma 375-8501 (JP)
(74) Representative: Green, Mark Charles
(86) International application number: PCT/JP2007/060280
(87) International publication number: WO 2007/138896

(56) References cited:
- JP-A- 06 022 816
- JP-A- 09 322 819
- JP-A- 2003 040 725
- JP-Y2- 63 030 335

## Description

### TECHNICAL FIELD

The present invention relates to a viscous cosmetic item, and more particularly, relates to a viscous cosmetic item in which a viscous cosmetic material is contained in a push-out container capable of withdrawing a content, wherein the viscous cosmetic item is provided with both a container shape capable of applying and manipulating the viscous cosmetic material effectively and a viscosity of viscous cosmetic material which is suitable to that shape, and the present invention also relates to a viscous cosmetic item in which a viscous cosmetic material prepared in a formulation for preventing a secondary contamination caused by microorganisms is contained in a containment part.

### BACKGROUND ART

Recently, a container-shaped viscous cosmetic item has been commercially available which contains a viscous cosmetic material in a cylinder-shaped containment part without using a conventional solid and bar-shaped cosmetic material and applies this cosmetic material on a part to which cosmetic material is applied, such as a lip, by means of an application member provided at the tip of a container, through a introduction hole portion communicated with the containment part. (For example, see JP-A-H09-322819.) Where a viscous cosmetic item using such a container is used, a container-shaped viscous cosmetic item has also been commercially available which contains a viscous cosmetic material in a cylinder-shaped containment part and ejects the viscous cosmetic material outward from an ejection port formed at the tip of the containment part by means of a mechanism for pushing up a member acting as a piston and arranged in this containment part, such as an internal block (a piston body, etc.) thereby conducting its application on a part to be applied, such as a lip. Furthermore, such a cosmetic item is a viscous cosmetic item which can control a viscous cosmetic material to be used or can also withdraw an extra viscous cosmetic material ejected on an application face into a containment part again by moving an internal block in the containment part up and down, thereby being capable of withdrawing a viscous cosmetic material remaining on the application face after use into the containment part again. However, such an ejection port formed at the tip of an application part of a container normally is provided at a closed state such that opening is conducted by means of the pressure of a viscous cosmetic material at a time when an internal block in a containment part is pushed up. Therefore, due to the shape of such an ejection port, there has been a problem that an extra viscous cosmetic material ejected on an application face is not sufficiently withdrawn into a containment part even though an internal block is moved down to the bottom part of a container.

Furthermore, an application face at the tip of an application part which face contacts a part on which cosmetic material is applied, such as a lip, has been in an inclined shape basically, but it has been formed with an ejection port having steps, has not been in a flat shape with respect to a part to be applied, and additionally, has been made from a plastic thereby having no flexibility and being hard, and has not had sufficient material or has not been properly shaped to conduct preferable application on a soft part on which cosmetic material is applied, such as a lip.

Moreover, operation of such a container of the viscous cosmetic item may have been difficult depending on the viscosity of a viscous cosmetic material used for the container. This operation is dependent on the rate or degree of moving up and down an internal block of an containment part, that is, the rotational speed or number of rotations of a rotator (such as an operation conversion part) that is moved up and down in conjunction with the internal block in a containment part which is provided on the bottom part of a container for containing a viscous cosmetic material, and adjustment of the rotational speed or number of rotations of the rotator has been difficult due to over-ejection outward from an ejection port formed at the tip of an application part or other ejection difficulty. For example, when conducting application on a part to which cosmetic material is applied, such as a lip, using a viscous cosmetic item containing a viscous cosmetic material with a high viscosity, ejection is not conducted from an ejection port formed at the tip of an application part even though the rotational speed or number of rotations of a rotator is increased considerably, and on the other hand, when conducting application using a viscous cosmetic item containing a viscous cosmetic material with a low viscosity, there have been problems such as over-ejection from an ejection port formed at the tip of an application part, liquid dripping from the ejection port caused even by a slight rotational operation, and a low retention of the viscous cosmetic material in a containment part whereby leakage from the ejection port may be caused depending on the rotational speed or number of rotations of a rotator when the tip portion of a container is directed downward, unless the operation is conducted with adjustment to significantly reduce the rotational speed or number of rotations of a rotator.

Furthermore, it has been found that such problems are associated with the shape of the inside of a moving container in which a viscous cosmetic material is present.

Therefore, it has been necessary to control the shape of a container and the viscosity of a viscous cosmetic material appropriately in order to retain the viscous cosmetic material in the container effectively, to conduct efficient ejection outward from an ejection port formed at the tip of an application part, and to withdraw again an extra viscous cosmetic material ejected on an application face into the container efficiently.

Furthermore, when a viscous cosmetic material remaining on an application face after use is withdrawn into a containment part again in such a viscous cosmetic item, the viscous cosmetic material contacting an application part contacts an unused viscous cosmetic material in the containment part, and thereby, the unused viscous cosmetic material in the containment part is easily contaminated by microorganisms attached to skin, lips or the like or ones capable of being digested by microorganisms, such as sweat, sebum, released pieces of each layer and food debris, which is unsanitary.

Meanwhile, paraben has been used commonly and frequently in order to provide a cosmetic material with an antiseptic effect for resistance to contamination caused by, for example, microorganisms such as bacteria, in the cosmetic material contacting a part to which cosmetic material is applied such as skin or a lip, and recently, dihydric alcohols such as dipropylene glycol have been used for antiseptic agents which replace paraben. (For example, see JP-A-H06-239731 and JP-A-2003-040725.) While dyhydric alcohols such as dipropylene glycol have been conventionally used as a moisture retention component for one of the raw materials of a cosmetic material, the effect thereof as an antiseptic agent has also been found and there is a possibility of having a more effective antiseptic effect than paraben depending on formulations, whereby its availability has been emphasized, its application on various cosmetic materials has been made, and for example, a formulation including paraben and a dihydric alcohol such as dipropylene glycol or a formulation including a dihydric alcohol such as dipropylene alcohol and including no paraben are prepared depending on formulations of viscous cosmetic materials.

Among such viscous cosmetic items using a push-out container capable of withdrawing a content, however, a viscous cosmetic item has not been present which has a viscosity of viscous cosmetic material as described above and uses a prepared viscous cosmetic material including a dihydric alcohol such as dipropylene glycol as an antiseptic agent for prevention of secondary contamination caused by microorganisms.

### DISCLOSURE OF THE INTENTION

### PROBLEMS TO BE SOLVED BY THE INVENTION

Therefore, the present invention has been made by taking the above-mentioned matters into consideration and an object of the present invention is to provide a viscous cosmetic item in which a viscous cosmetic material is contained in a push-out container capable of withdrawing a content, wherein the viscous cosmetic item is provided with both a container shape capable of applying and controlling the viscous cosmetic material effectively and a viscosity of viscous cosmetic material which is suitable to that shape.

More particularly, there is provided a viscous cosmetic item characterized in that a container has an application part with a material and shape for applying a viscous cosmetic material effectively and a pipe diameter which is easy to handle even for viscous cosmetic materials with different viscosities and a viscous cosmetic material in the container which is suitable to that pipe diameter has a viscosity for moving in the container efficiently and being retained in the container effectively.

Furthermore, another object of the present invention is to provide a viscous cosmetic item using a prepared viscous cosmetic material including a dihydric alcohol such as dipropylene glycol as an antiseptic agent for avoiding the possibility of contamination caused by, for example, microorganisms, in addition to the above-mentioned features.

### MEANS FOR SOLVING THE PROBLEM

The inventors have actively investigated and consequently have developed a viscous cosmetic item of the present invention, by a viscous cosmetic item in which a viscous cosmetic material is contained in a push-out container capable of withdrawing a content, wherein the container has an application part with a material and shape for applying a viscous cosmetic material effectively and a pipe diameter which is easy to handle even for viscous cosmetic materials with different viscosities and a viscous cosmetic material in the container which is suitable to that pipe diameter has a viscosity for moving in the container efficiently and being retained in the container effectively and includes a dihydric alcohol, such as dipropylene alcohol, as an antiseptic agent for avoiding the possibility of contamination caused by, for example, microorganisms.

That is, the present invention is achieved by a viscous cosmetic item comprising a viscous cosmetic material being contained in a push-out container capable of withdrawing a content and forming a cylinder-shaped containment part, an application part being provided at a tip of this container, and an internal block is installed in the containment part such that a rotator located on a part of the container is rotated in one direction thereby pressurizing the viscous cosmetic material contained in the containment part to make it possible to push the viscous cosmetic material outward from an ejection port provided on an application face of the application part and the rotator is rotated in a reverse direction thereby making it possible to withdraw an extra viscous cosmetic material on the application part from the ejection port of the application face into the containment part, characterized in that the viscous cosmetic item comprises the application part being composed of an elastic material, the application face being an inclined plane face inclined with respect to a longitudinal axis of a container body, a cylinder-shaped viscous cosmetic material supply pipe of the container having a diameter of 1.7 to 2.2 mm, an ejection port being to eject the viscous cosmetic material outward and communicated therewith being provided on the application face at an open state, and the viscous cosmetic material contained in the containment part of the container having a viscosity of 19000 mPa·s or greater at a shear rate of 0 to 5 sec⁻¹ and having a viscosity of 11000 mPa·s or less at a shear rate of 40 sec⁻¹.

According to the invention as described above, the effect of the application of a viscous cosmetic material in use can be enhanced by the shape of an application face composed of an elastic material, for example, a resin rubber, and formed in a container, being an inclined plane face inclined with respect to a longitudinal axis of a container body, and a viscous cosmetic material remaining on an application part after use can be readily withdrawn into the container by an ejection port at an open state being to eject the viscous cosmetic material outward and communicated with a cylinder-shaped viscous cosmetic material supply pipe with a diameter of 1.7 to 2.2 mm of the container being provided on the application face. Furthermore, only a suitable amount of a viscous cosmetic material for makeup can be used without waste because a viscous cosmetic material contained in a containment part of the container has a viscosity of 19000 mPa·s or greater at a shear rate of 0 to 5 sec⁻¹ and has a viscosity of 11000 mPa·s or less at a shear rate of 40 sec⁻¹ whereby the viscous cosmetic material can move in the container efficiently and the viscous cosmetic material can be retained in the container effectively, depending on the rotation of a rotator in one direction or a reverse direction relative thereto which is provided on a part of container including a containment part (that is, depending on suitable up-and-down movement of an internal block arranged in the containment part).

Moreover, a viscous cosmetic item of the present invention is characterized in that the viscous cosmetic material includes 0.2 wt% or more of dipropylene glycol.

According to the invention as described above, not only a suitable amount of a viscous cosmetic material for makeup can only be used without waste by rotating a rotator provided on a part of the container in one direction or a reverse direction relative thereto thereby moving up and down an internal block arranged in the containment part and by containing a viscous cosmetic material prepared in a formulation for prevention of secondary contamination cased by microorganisms in a cylinder-shaped containment part in which the content of included dipropylene glycol is 0.2 wt% or more, in addition to the viscosities as described above, but also an application face after use does not become unsanitary and secondary contamination caused by microorganisms can be prevented effectively due to the contamination prevention effect of 0.2 wt% or more of an included dihydric alcohol such as dipropylene glycol, thereby having an effective antiseptic effect against, for example, contamination, also in the preservation thereof and being capable of keeping the viscous cosmetic material in a good state, particularly even when a viscous cosmetic material remaining on an application face after makeup is contained in the containment part again by rotating the rotator in the reverse direction.

### ADVANTAGEOUS EFFECT OF THE INVENTION

Therefore, in a viscous cosmetic item according to the present invention in which a viscous cosmetic material is contained in a push-out container capable of withdrawing a content, the effect of application of a viscous cosmetic material in use can be enhanced by the shape of an application face composed of an elastic material, such as a resin rubber, and formed in a container being an inclined plane face inclined with respect to a longitudinal axis of a container body and further a viscous cosmetic material remaining on an application part after use can be readily withdrawn into the container by an ejection port at an open state being to eject the viscous cosmetic material outward and communicated with a cylinder-shaped viscous cosmetic material supply pipe with a diameter of 1.7 to 2.2 mm of the container being provided on the application face. In addition, a viscous cosmetic material can move in a container efficiently and can be retained in the container effectively by a viscous cosmetic material being suitable to that pipe diameter, having a viscosity of 19000 mPa·s or greater at a shear rate of 0 to 5 sec⁻¹ and having a viscosity of 11000 mPa·s or less at a shear rate of 40 sec⁻¹ in the container, and further, only a suitable amount of the viscous cosmetic material for makeup can be used without waste by rotating a rotator provided on a part of the container including a containment part in one direction or a reverse direction relative thereto thereby moving up and down an internal block arranged in the containment part.

Moreover, an application face after use does not become unsanitary by forming a viscous cosmetic item in which a prepared viscous cosmetic material in which the content of a dihydric alcohol such as dipropylene glycol is 0.2 wt% or more is contained in a containment part, in addition to the above-mentioned moderate viscosities, particularly even when a cosmetic material remaining on the application face after makeup is contained in the containment part again by rotating the rotator in the reverse direction, and it becomes possible to provide effective prevention of secondary contamination caused by microorganisms, to have antiseptic effect against, for example, contamination, also in preservation, to keep an unused viscous cosmetic material in a good state, and to prevent degradation of a product, due to the effect of 0.2 wt% or more of an included dihydric alcohol such as dipropylene glycol.

### BRIEF DESCRIPTION OF THE DRAWINGS

FIG. 1 is a cross-section diagram of a viscous cosmetic item of the present invention.
FIG. 2 is an enlarged cross-section diagram of a container tip portion of a viscous cosmetic item of the present invention.
FIG. 3 is an enlarged cross-section diagram of another container tip portion of a viscous cosmetic item of the present invention.
FIG. 4 is an enlarged cross-section diagram of yet another container tip portion of a viscous cosmetic item of the present invention.
FIG. 5 is a cross-section diagram of a viscous cosmetic item of the present invention.
FIG. 6 is a set of perspective view and longitudinal section view of an application body of container of a viscous cosmetic item of the present invention.
FIG. 7 is a ser of longitudinal section view, perspective view and transverse section view along the C-C line of FIG. 5, of a rotator of the viscous cosmetic item shown in FIG. 5.
FIG. 8 is a cross-section diagram of another embodiment of a viscous cosmetic item of the present invention.
FIG. 9 is a graph showing the relationship between the viscosity of a viscous cosmetic material and shear rate for a viscous cosmetic item of the present invention.

### EXPLANATION OF LETTERS OR NUMERALS

1: Push-out container capable of withdrawing a content
2: Body
2a: Small diameter part
2b: Application liquid containment space (storage tank)
3: Leading axle
3a: Fitting concave part
3b: Flange part
3c: Rib
4: Application liquid
6: Liquid pressurizing mechanism (liquid pressurizing means)
7: Cap
10: Application body
10a: Application part
12: Pipe joint
12a: Through-hole
13: Application liquid supply pipe
20: Viscous cosmetic item
21: Tapered part
24: Communicating path (slit)
24a: Ejection port
25: Planar part (temporary liquid retaining part)
31: Rotator
32: Screw stock
34: Retention member
34a: Larger diameter part
34b: Smaller diameter part
35: Piston body (internal block)
36: Outer cylinder cap
37: Inner cylinder material
38: Fitting part
39: Engaging part
41: Liquid retaining part
44: Comb part
45: Communicating path
45a: Ejection port
47: Liquid retaining part
50: Viscous cosmetic item
51: Cap
52: Storage tank
53: Liquid depressurizing mechanism (liquid depressurizing means)
55: Operation conversion part (rotator)
56: Outer cylinder cap
57: Inner cylinder material
58: Elastic structure
58a: Protrusion
59: Elastic structure
59a: Protrusion
60: Flange part
61: Fitting part
R: Clockwise rotation direction (pressurization direction)

### BEST MODE FOR CARRYING OUT THE INVENTION

Specific examples implemented according to the present invention will be described in more detail below but the present invention is not limited to these specific examples. Additionally, it is possible for the details thereof to be various configurations unless departing from the scope of the present invention.

A viscous cosmetic item according to the present invention is a viscous cosmetic item including a viscous cosmetic material being contained in a push-out container capable of withdrawing a content and forming a cylinder-shaped containment part, an application part being provided at the tip of this container, and an internal block being installed in the containment part such that a rotator located on a part of the container is rotated in one direction thereby pressurizing the viscous cosmetic material contained in the containment part to enable the viscous cosmetic material to push outward from the ejection port provided on the application face of the application part and the rotator is rotated in a reverse direction thereby enabling an extra viscous cosmetic material on the application part to withdraw from an ejection port of an application face into the containment part, wherein the viscous cosmetic item is characterized by the application part for applying the viscous cosmetic material being provided at the tip portion of the container, the application part being composed of an elastic material, the application face being an inclined plane face inclined with respect to the longitudinal axis of a container body, the pipe diameter of a cylinder-shaped viscous cosmetic supply pipe in the container being 1.7 to 2.2 mm, an ejection port being communicated therewith, being to eject the viscous cosmetic material outward and being provided on the application face at an open state, characterized by the viscous cosmetic material contained in the containment part of the container having a viscosity of 19000 mPa·s or greater at a shear rate of 0 to 5 sec⁻¹ and having a viscosity of 11000 mPa·s or less at a shear rate of 40 sec⁻¹, and further characterized by a prepared viscous cosmetic material including 0.2 wt% or more of a dihydric alcohol such as dipropylene glycol as an antiseptic agent being contained in the push-out container capable of withdrawing a content.

First, the configuration of a container for a viscous cosmetic item according to the present invention will be described below. A container according to the present invention is characterized that an application part at the tip of the container to apply on a part which a viscous cosmetic material is applied contained in a containment part provided in the container is provided to have a material and shape which are easy to apply the viscous cosmetic material efficiently and easy to handle at the time of application, and is further characterized by having a shape of the container which is operable so that the viscous cosmetic material is easy to apply effectively.

As will be described in more detail, the present invention is characterized by the application part at the tip of the container being composed of an elastic material, an application face of the application part being an inclined plane face inclined with respect to the longitudinal axis of a container body, the pipe diameter of a cylinder-shaped viscous cosmetic material supply pipe in the container being 1.7 to 2.2 mm, and an ejection port communicating therewith, to eject the viscous cosmetic material outward and being provided on the application face at an open state.

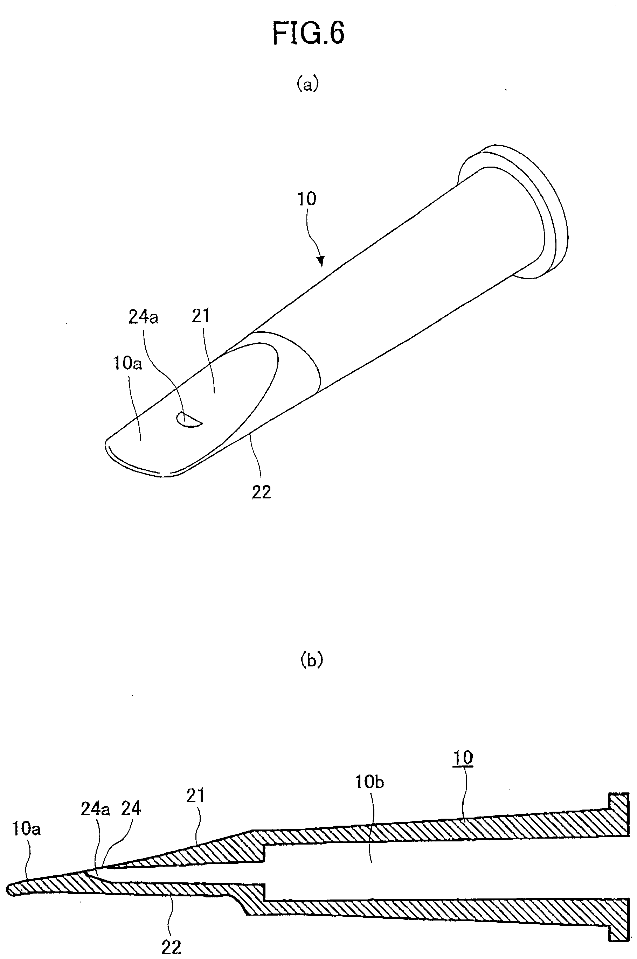

For one configuration of an application part provided on the viscous cosmetic item according to the present invention, the configuration of an application part composed of an elastic material such as a resin rubber, for example, a urethane rubber is, as shown in FIG. 6, a shape provided by inclining and cutting the tip of a cylinder-shaped container, that is, a configuration of the application face being an inclined plane face inclined with respect to the longitudinal axis of a container body, an upper and outer face being an application face and also an application part, a viscous cosmetic material supply pipe with a pipe diameter of 1.7 to 2.2 mm, and an ejection port communicating therewith, to eject the viscous cosmetic material outward and being provided on the application face at an open state. Due to this configuration, the application part has a large surface area to contact any part to be applied such as skin or a lip flexibly and suitably, for example, in order to fix on a part to be applied such as skin or a lip, and a viscous cosmetic material ejected from the ejection port on the application face extends on the application face, so that application can easily and more simply be conducted for any part to which cosmetic material is applied such as skin or a lip. Furthermore, a viscous cosmetic material supply pipe with a pipe diameter of 1.7 to 2.2 mm communicates with an ejection port formed on the application face, whereby the viscous cosmetic material efficiently moves in the viscous cosmetic material supply pipe of the container and an amount of a viscous cosmetic material which is necessary for use can be prepared and can be used effectively without waste.

While the parts other than the application part at the tip of the push-out container capable of withdrawing a content or viscous cosmetic material according to the present invention have been publicly-known and therefore are not particularly described in detail, the container for the present invention is provided with, for example, an internal block such as a piston body in a containment part and that internal block from a rotator provided as a bottom part of the container is connected to a mechanism for moving in the manner of piston in the container, according to a preferred embodiment of the present invention. When this rotator is rotated in the clockwise or counter-clockwise direction, the internal block is moved up or down so that the viscous cosmetic material is allowed to eject from or be withdrawn into the application part. This rotator serves to switch the operations of the container and therefore is also referred to as an operation conversion part. Therefore, the viscous cosmetic material for the present invention is contained in a region from the top of the containment part to the internal block, and an amount of a viscous cosmetic material which is necessary for use can be controlled and can be used effectively without waste. According to the present embodiment, the rotator is provided on the bottom part of the container and is actuated to rotate in the clockwise or counter-clockwise direction but may be provided on any portion of the container as a part of the container and the actuation thereof may also be conducted by means of any actuators, and the configuration of the push-out container capable of withdrawing a content or viscous cosmetic material according to the present invention is not particularly limited as long as it is within scope of the present invention and is allowed to be various configurations.

Next, a viscous cosmetic material for the viscous cosmetic item according to the present invention will be described. A viscous cosmetic material contained in a containment part provided in a container of the viscous cosmetic item according to the present invention is a viscous cosmetic material suitable to a viscous cosmetic material supply pipe with a pipe diameter of 1.7 to 2.2 mm which is characterized by moving in the containment part of the container efficiently and being prepared with a moderate viscosity for effective retention in the containment part of the container.

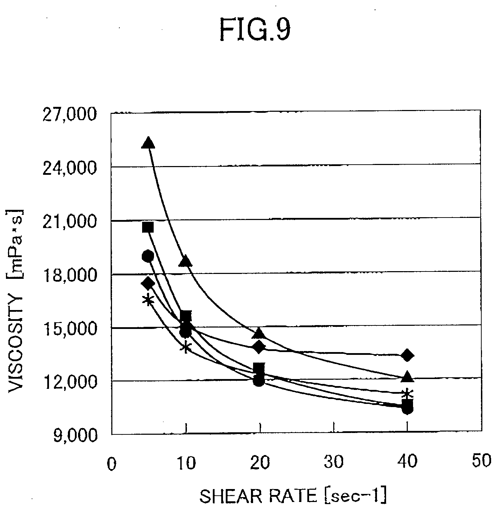

According to a preferred embodiment of the present invention, a viscous cosmetic material contained in a containment part of the container for the viscous cosmetic item according to the present invention has a viscosity of 19000 mPa·s or greater at rest, that is, at a shear rate of 0 to 5 sec⁻¹, and has a viscosity of 11000 mPa·s or less at a higher shear rate, that is, at a shear rate of 40 sec⁻¹. This indicates the relationship between the viscosity and the shear rate, as shown in curved lines with squares and curved lines with circles in FIG. 9. If the viscosity at a shear rate in use, that is, a shear rate of 40 sec⁻¹, is greater than 11000 mPa·s, the viscosity of a viscous cosmetic material is so high that the movement in the containment part is difficult and accordingly the ejection from an ejection port is also difficult even if the rotational speed of the rotator provided in the bottom portion of the container is increased or the number of rotations is increased. Also, if the viscosity at rest or at a shear rate of 0 to 5 sec⁻¹ is less than 19000 mPa·s, the viscosity of the viscous cosmetic material so small that the retention in the containment part is difficult, for example, spilling out of the ejection port communicated with the containment part occurs even if the rotator provided on the bottom portion of the container is rotated slightly.

The viscous cosmetic material for the viscous cosmetic item according to the present invention includes 2 wt% to 3 wt% of a dextrin palmitate as a setting agent and 2.5 wt% to 3.0 wt% of a silylated anhydrous silicic acid as a thickening agent so as to keep a suitable viscosity.

Furthermore, the viscous cosmetic material for the viscous cosmetic item according to the present invention has the above-described viscosities, whereby there is no concern about the leakage or liquid dripping of the viscous cosmetic material even if conducting the storage at a high temperature, and it is possible to maintain clean and comfortable use, to obtain light and smooth application when the viscous cosmetic material is applied on a part to which the material is to be applied, to conduct uniform application on any part to which the material is to be applied such as skin or a lip, and to obtain a good finish with less blur on an application part.

Moreover, according to another preferred embodiment of the present invention, a viscous cosmetic material for the viscous cosmetic item of the present invention is characterized by including 0.2 wt% or more of a dihydric alcohol such as dipropylene glycol. Thereby, the viscous cosmetic material for the viscous cosmetic item of the present invention is a viscous cosmetic material such as viscous lip rouge (lip gloss) in a formulation for prevention of secondary contamination caused by microorganisms, and contamination of unused viscous cosmetic material which is caused by microorganisms can be prevented and further degradation can be prevented by containing this viscous cosmetic material in the push-out container forming a cylinder-shaped containment part and being capable of withdrawing a content and by rotating the rotator in the reverse direction, even if an extra viscous cosmetic material on an application part is withdrawn from the ejection port on the application face into the containment part. Additionally, dihydric alcohols such as 1,3-butanediol, isopropylene glycol, propylene glycol, pentanediol, and hexylene glycol may have a similar effect depending on a formulation of the viscous cosmetic material for the viscous cosmetic item of the present invention. Therefore, an antiseptic agent used in the viscous cosmetic material for the viscous cosmetic item of the present invention is not necessarily limited to dipropylene glycol but also one selected from the group of these dihydric alcohols can be used depending on a formulation of the viscous cosmetic material.

Due to these characteristics, the viscous cosmetic material for the viscous cosmetic item of the present invention does not only have a viscosity suitable to a container having a pipe diameter of 1.7 to 2.2 mm but also has antiseptic effect for preventing secondary contamination caused by microorganisms, by including 0.2 wt% or more of an antiseptic agent selected from the group of dihydric alcohols such as dipropylene glycol depending on a formulation of the viscous cosmetic material.

Additionally, the detailed composition of a viscous cosmetic material for the viscous cosmetic item of the present invention will be illustrated in the examples described bellow, wherein dihydric alcohols such as dipropylene glycol for a setting agent, a thickening agent and an anti-septic agent in order to provide an appropriate viscosity have been publicly-known. Furthermore, for other components of the viscous cosmetic material for the present invention, an oil component such as a deodorant polybutene, vaseline, a liquid paraffin, triisostearin, diisostearyl malate, castor oil, or another liquid oil component is used and a coloring material such as Red No. 202 or safflower red is used, wherein these have been all publicly-known. Because a method for manufacturing the viscous cosmetic material for the present invention has also been publicly-known, it is possible for a person skilled in the art to conduct its formation easily according to a conventional method for manufacturing a viscous cosmetic material.

The viscous cosmetic material for the viscous cosmetic item of the present embodiment is viscous cosmetic rouge but its configuration is not limited and may be any configuration as long as it is used as a cosmetic item and does not depart from the scope of the present invention.

### Examples

The present invention will be illustrated with reference to some examples below but the present invention is not limited by these examples.

A push-out container capable of withdrawing a content in the viscous cosmetic item according to the present invention will be described.

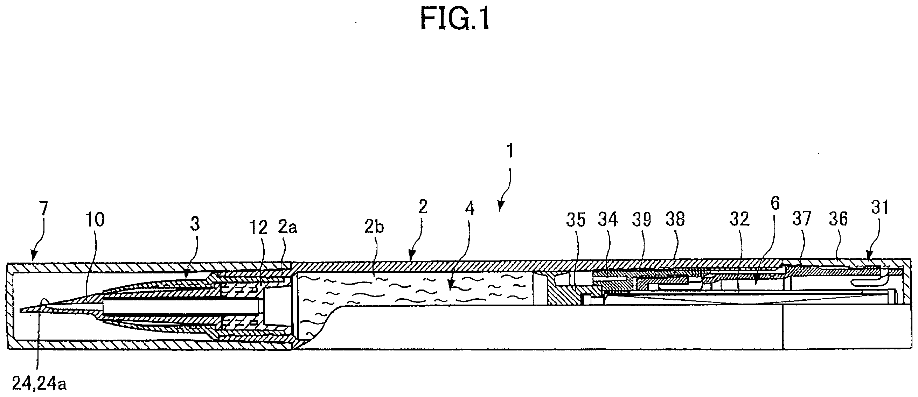

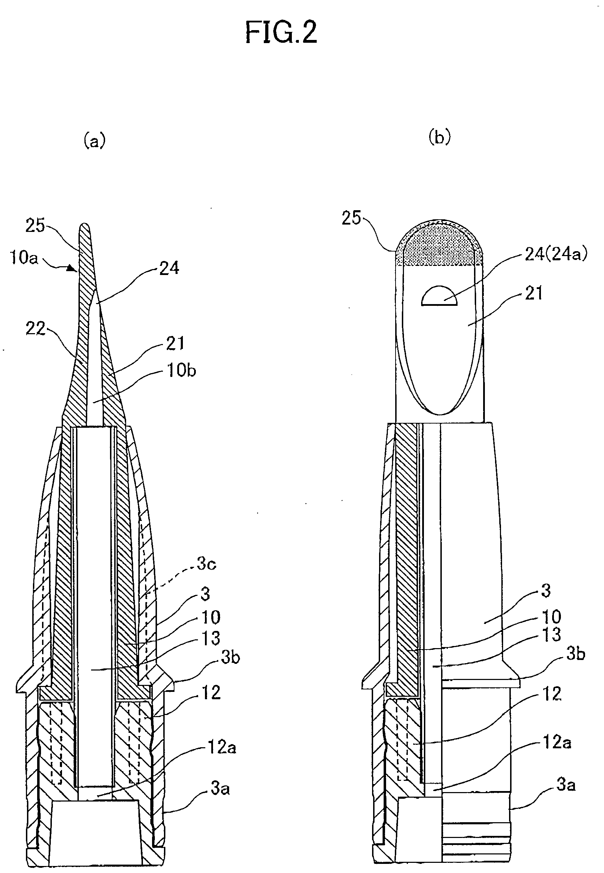

First, FIG. 1 illustrates an embodiment thereof in the case where a viscous cosmetic material for the present invention is pushed out of a container and FIGS. 2 to 4 illustrate various configurations of an application body composing an application part.

As shown in FIG. 1, a viscous cosmetic item 1 according to a first embodiment is provided with a liquid pressurizing mechanism (liquid pressurizing means) 6 for pressurizing a viscous cosmetic material 4 in a body 2 and a piston body 35 as an internal block is provided at the tip thereof. That is, the piston body 35 is configured such that the viscous cosmetic material 4 is supplied to an application body 10 by a pressure of the liquid pressurizing mechanism 6.

The application body 10 of the viscous cosmetic item 1 is composed of an elastic material and a communicating path 24 communicating with the inside of the body 2 and exterior thereof is formed for the application body 10. An application part 10a at the tip portion of the application body 10 forms an application face integrally with a tapered part 21 and an ejection port 24a for ejecting the viscous cosmetic material 4 from the communicating path 24 is provided near the center of that application face.

Furthermore, as shown in FIG. 1, the viscous cosmetic item 1 has the body 2 being an outer cylinder, a leading axle, the viscous cosmetic material 4, the liquid pressurizing mechanism 6, and a cap 7, as main members.

The body 2 generally has a hollow cylinder shape and a small diameter part 2a having an outer diameter that is generally identical to the inner diameter of a tapered cap 7 is formed at the tip portion thereof, wherein the cap 7 is attachably and detachably fitted with that small diameter part 2a.

The rear anchor of the leading axle 3 is fitted fluid-tightly in the opening of the small diameter part 2a of the body 2 and the liquid pressurizing mechanism 6 is arranged at the back end of the body 2 wherein the piston body 35 of the liquid pressurizing mechanism 6 is provided slidably while closely contacting the inner wall of the opening at the back end of the body 2.

Therefore, the part surrounded by the inside of the body 2, the back end portion of the leading axle 3, and the piston body 35 is formed as a viscous cosmetic material containment space (storage tank) 2b for the viscous cosmetic material 4.

The liquid pressurizing mechanism 6 is composed of a rotator (that is also referred to as a rotation operation member) 31, a screw stock 32 (push element), a retention member 34 for the screw stock 32, and the above-mentioned piston body 35, as main members.

The rotator 31 is composed of an outer cylinder cap 36 and an inner cylinder member 37 which are connected not to be rotatable with respect to each other, and the rotator 31 is provided to be entirely rotatable with respect to the body 2.

The retention member 34 is composed of a ring-shaped member and is attached to the body 2 so as not to be rotatable. A ratchet is formed on a fitting part 38 of both the retention member 34 and the rotator 31 (peripheral surface in front of the inner cylinder member 37) and the rotator 31 is rotation-regulated to rotate in only one direction with respect to the retention member 34 (the body 2 on which it is fixed). Additionally, when a certain or greater rotary force is applied in the one direction, it is possible to provide a torque limiter function for releasing that regulation and making it rotatable.

A male screw is formed on the outer periphery of the screw stock 32 and that male screw is engaged with a female screw formed in a hole of the center portion of the retention member 34. Furthermore, the peripheral surface portion of the screw stock 32 has an anisotropic cam shape in the cross-sectional view (for example, has a generally elliptical shape in the cross-sectional view due to a notch formed on an edge portion in the radial direction) and an engaging part 39 of the inner cylinder member 37 of the rotator 31 (in front of the fitting part 38) has an anisotropic cam shape, wherein the shape of its center hole corresponds to the peripheral shape of the screw stock 32. The above-mentioned screw stock 32 is inserted in and penetrates through the center hole of the engaging part 39, whereby the screw stock 32 is engaged with the inner cylinder member 37 (via the engaging part 39) to be slidable and in the axial direction, and not to be relatively rotatable.

The tip portion of the screw stock 32 is connected to the piston body 35, and when the rotator 31 is rotated in a predetermined direction, the screw stock 32 moves forward, toward the tip portion of the body 2, via the retention member 34, whereby the piston body 35 is moved toward the tip portion of the piston body 35 to pressurize the viscous cosmetic material 4.

As shown in FIG. 2, a leading axle 3 is formed to provide a taper-shaped cylinder structure whose diameter decreases toward the tip thereof, wherein an application body 10 is contained in internal space provide in its vicinity on the condition that its tip portion protrudes and a body 2 is fixed on the containment condition. As described below, the application body 10 has a shape becoming more flattened and thin toward the tip thereof.

A ring-shaped fitting concave part 3a is formed on the rear perimeter of the leading axle 3. The fitting concave part 3a is pressed and fitted into a ring-shaped fitting convex part (not shown in the figure) formed on the inner face of a small diameter part 2a in the body 2, thereby preventing the leading axle 3 from detaching from the body 2.

Furthermore, a flange part 3b is formed on the peripheral portion of the leading axle 3 and the flange part 3b contacts the front end portion of the small diameter part 2a. On the inner face of this leading axle 3, a plurality of ribs 3c extending outward in its axis directions are formed on the inner face at even intervals and a flange-like portion having an increased diameter at the back end of the application body 10 is pinched by the back end faces of the ribs 3c and the front end face of a pipe joint 12, so that the application body 10 is pinched and fixed in the inside of the leading axle 3.

The application body 10 is composed of an elastic material and is supported by the pipe joint 12 and a viscous cosmetic material supply pipe 13. Furthermore, as described in detail below, the diameter of the viscous cosmetic material supply pipe 13 is preferably 1. 7 to 2.2 mm. The viscous cosmetic material supply pipe 13 is fitted, inserted and fixed in a central through-hole 12a of the pipe joint 12, and is inserted from the rear end portion of a hole part 10b provided on the application body 10 to the middle portion of the hole part which has a large diameter (the tip portion of the leading axle 3), thereby also serving as a liquid leakage suppression mechanism.

Both faces of the application part 10 are tapered parts 21, 22 to have a flat and tapered shape. The tapered part 21 on the top face is integrated with an application face and is formed on a plane. An application part 10a of the application body 10 is formed of, particularly, a urethane rubber.

A communication path 24 communicates with the hole part 10b of the application part 10 and further communicates with an application liquid supply pipe 13. An ejection port 24 of the communication path 24 is at an open state.

In the present invention, the entirety of the application part 10 is composed of an elastic material and is composed of a urethane rubber similarly to the application part 10a. For the material of the application part 10, elastic materials such as rubbers and elastomers can be provided.

### For example,

(1) for rubbers, there are provided NBRs, silicon rubbers, EPDMs, fluorosilicon rubbers, fluorine rubbers, urethane rubbers, natural rubbers, chloroprene rubbers, butadiene rubbers, butyl rubbers, and the like.
(2) for elastomers, there are provided styrene-based elastomers, vinyl chloride-based elastomers, olefin-based elastomers, polyester-based elastomers, polyamide-based elastomers, urethane-based elastomers, and the like.
(3) For closed-cell substances, there are provided polyethylene foams, polyvinyl chloride foams, polystyrene foams, and the like.

As shown in FIG. 2, the application part 10a is formed as a plane part 25 whose application part is smooth. A viscous cosmetic material 4 ejected from the ejection port 24a of the communication path 24 is retained on the plane part 25 at the tip side of the ejection port 24.

In such a configured viscous cosmetic item 1, on its condition, the viscous cosmetic material 4 fills in the body 2, in the pipe joint 12 of the leading axle 3, in the viscous cosmetic material supply pipe 13 and in the hole part 10b of the application body 10.

In use, a rotator 31 of a liquid pressurizing mechanism 6 is rotated. Due to the rotation of the rotator 31, a screw stock 32 goes forward toward its tip via a drive transmission of an engaging part 39. Thereby, a piston body 35 goes forward toward the tip to pressurize the viscous cosmetic material 4. A predetermined amount of the viscous cosmetic material 4 is ejected from the ejection port 24a by means of pressurization of the viscous cosmetic material 4.

The ejected viscous cosmetic material 4 is temporarily retained on the plane part 25 extending from the ejection port 24 to the tip, and is introduced and applied on a soft face to which the cosmetic material is to be applied, such as skin.

Furthermore, the liquid pressurizing mechanism 6 utilizes a ratchet and therefore has a mechanism for rotating the rotator 31 by a predetermined amount by one operation of the rotator 31 to push out the piston body 35 by a predetermined amount for every operation. As described below, the viscous cosmetic material can be supplied accurately on the application part 10a, that is, on the plane part 25, by a desired and predetermined amount, also due to the viscosity of the viscous cosmetic material 4 which is suitable to an application liquid supply pipe with a diameter of 2 mm.

Moreover, dripping of the viscous cosmetic material 4 to be ejected, which is caused by rapid ejection thereof or the like, can be prevented by providing a plane part (liquid retaining part) 25 for temporarily retaining the ejected viscous cosmetic material 4 on the application part 10a, at the ejection port 24a of the communication path 24.

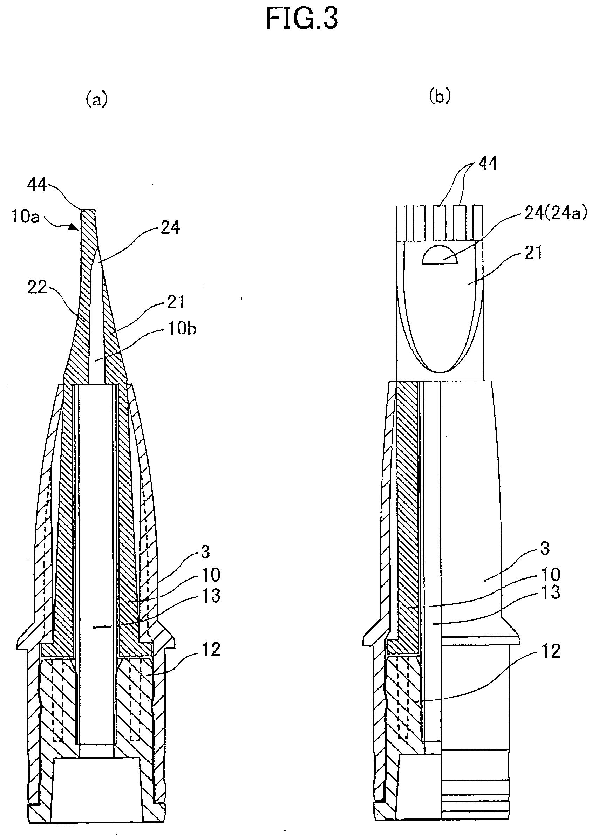

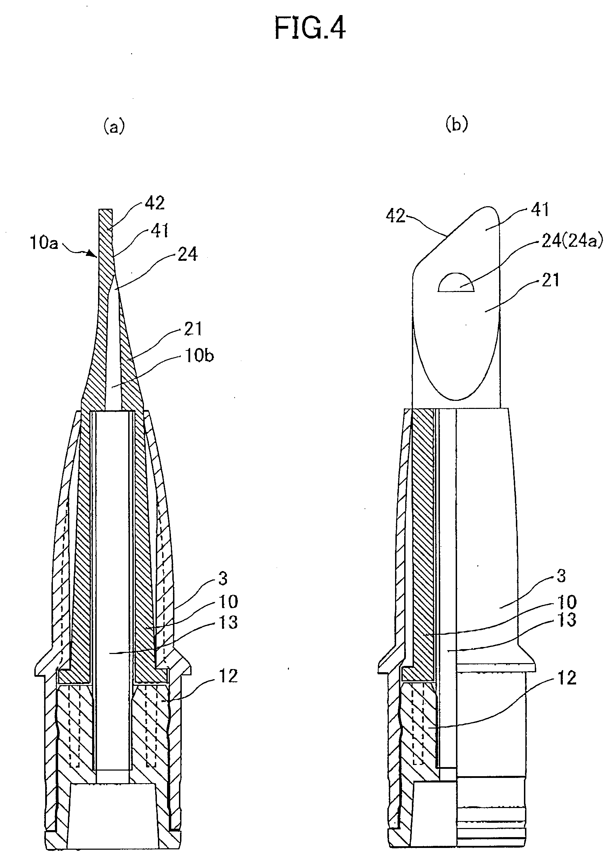

FIG. 3 and FIG. 4 are diagrams showing variations of an application part of an application body for the viscous cosmetic item of FIG. 1.

Similar parts or analog parts of viscous cosmetic items shown in FIG. 3 and FIG. 4 will be provided with the identical reference numerals and their detailed descriptions will be omitted because parts other than their application bodies 10 generally have structures similar to the structure of the leading axle of FIG. 2.

As shown in FIG. 3, a comb part 44 is further formed toward the tip from an ejection port 24a of a communication path 24, which is present on an application face formed in a plane shape and integral with the tapered part of the application part 10.

The viscous cosmetic material 4 ejected from the ejection port 24a temporarily adheres to the comb part 44 or is retained by means of the surface tension of that liquid and the comb 44 is a liquid retaining part for temporary retention and the comb 44 plays the central role of the application part 10a.

As shown in FIG. 4, the tip of a temporary liquid retaining part 41, which further extends to the tip from an ejection port 24a of a communication path 24 present on an application face formed in a plane shape and integral with a tapered part of an application body 10, has a knife shape formed on an acute angle part 42. Due to use of such a shape, a viscous cosmetic material 4 can be applied accurately along the profile of a lip or the like while temporary liquid retention is allowed.

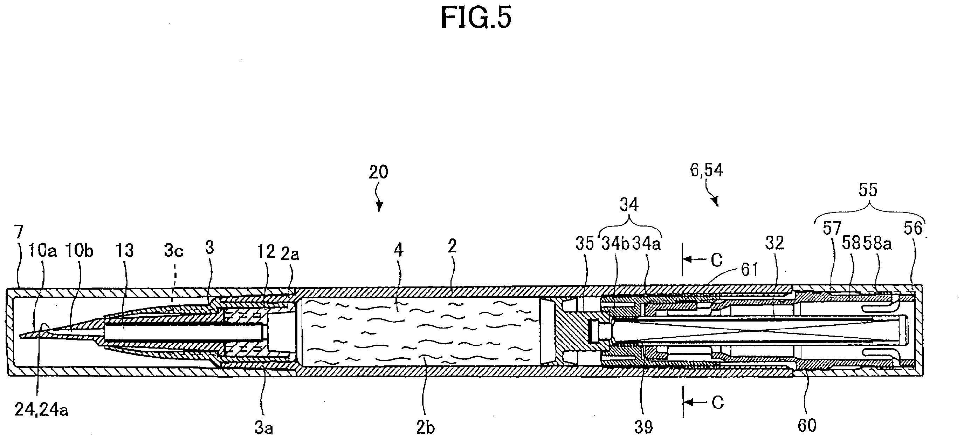

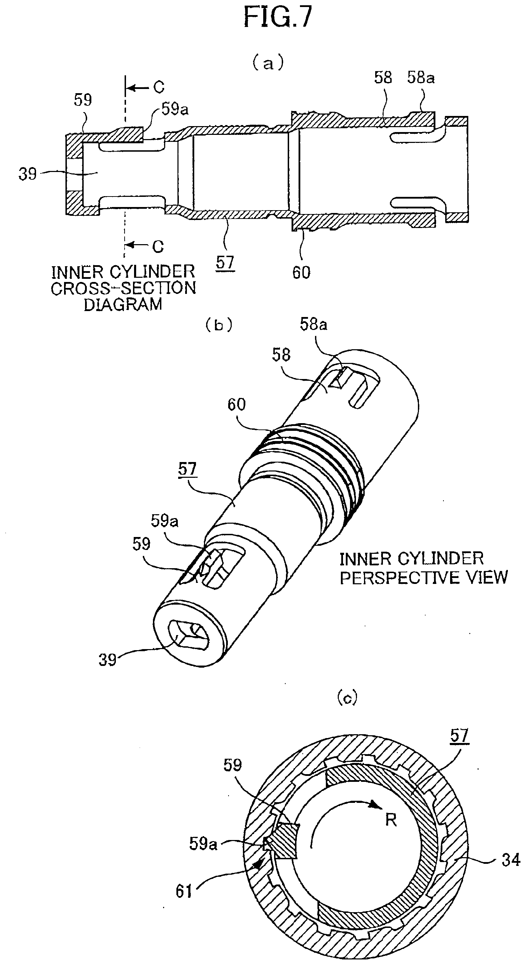

FIG. 5 is a longitudinal sectional view showing the entirety of a viscous cosmetic item according to an embodiment of the present invention which contains a viscous cosmetic material in a push-out container capable of withdrawing a content. FIG. 6 is a diagram illustrating the details of its application part. FIG. 7 is a diagram illustrating the details of a rotational dynamic body.

A viscous cosmetic item 20 shown in FIG. 5 generally has a structure similar to that of the viscous cosmetic item according to the first embodiment of the present invention as shown in FIG. 1 and FIG. 2 except the structure of its liquid depressurizing mechanism (liquid depressurizing means) 54, and therefore, identical reference numerals are provided to similar parts or analogous parts while their detailed descriptions are omitted.

As shown in FIG. 5, a viscous cosmetic item 20 is provided with a liquid depressurizing mechanism 54 for depressurizing a viscous cosmetic material 4 inside a body 2.

A liquid pressurizing mechanism 6 and the liquid depressurizing mechanism 54 have piston bodies (pressure application parts) 35 for pressurizing or depressurizing a viscous cosmetic material 4, which serve as internal blocks moving forward or backward in a viscous cosmetic material containment space 2b inside the body 2, and rotators 55 (referred to as an operation conversion part, below, in view of its functional purpose) for converting a user's rotational operation for rotators 31 facing the outside of the body 2 into the forward movement operation or backward movement operation of the piston bodies 35.

Specifically, a viscous cosmetic item 20 shown in FIG. 5 is composed of main members including a body 2 being an outer cylinder, a leading axle 3, a viscous cosmetic material 4, a liquid pressurizing mechanism 6, a liquid depressurizing mechanism 54, an operation conversion part 55, and a cap 7, as shown in FIGS. 6 and 7, and these configurations of the liquid depressurizing mechanism 54 and operation conversion part 55 are different from the only one liquid pressurizing mechanism 6 of the viscous cosmetic item 1, according to the first embodiment as shown in FIG. 1.

An application body 10 uses that shown in FIG. 6 which has a structure similar to that shown in FIG. 2. That is, in the application body 10, its exposed tip portion protruding from the head of the leading axle 3 has a flat and tapered shape at double-faced taper parts 21, 22, as viewed obliquely in FIG. 6 (a) and viewed in the cross section of each part in FIG. 6(b). The taper part 21 on its upper face is formed integrally with an application face and in a planar shape and an application part 10a of the application body 10 is further formed to be in a direction from an ejection port 24a toward the tip. For the application body 10, those embodiments as shown in FIGS. 3 and 4 can be used besides those embodiments shown in FIGS. 2 and 6.

As shown in FIG. 5, the liquid pressurizing mechanism 6 and the liquid depressurizing mechanism 54 which are provided integrally are arranged at the back end of the body 2 in the viscous cosmetic item 20, and piston bodies 35 of the liquid pressurizing mechanism 6 and liquid depressurizing mechanism 54 are provided slidably while closely contacting the inner wall of an opening at the back end of the body 2.

The liquid pressurizing mechanism 6 and liquid depressurizing mechanism 54 are composed of main members including operation conversion parts 55, screw stocks 32 (push elements), retention members 34 for the screw stock, and the piston bodies 35 as described above.

The operation conversion part 55 is composed of an outer cylinder cap 56 and inner cylinder member (also referred to as "a feeding body") 57 which are joined to each other in order not to be relatively rotatable normally and to be relatively rotatable by means of a critical or greater rotary force, and the entirety of the operation conversion part 55 is provided to be rotatable with respect to the body 2.

For details, the inner cylinder 57 is a generally cylinder shape with diameters that stepwise increase as one moves forward from a front part (57f) through a central part (57c) to a back part (57r), which is provided with notches at some positions, as shown in FIG. 7.

The inner cylinder member 57 is formed with both a cantilever-like elastic structure 58 provided with elasticity, at least, at the outer side of its radial direction, by means of a U-shaped notch at the side face part of its back portion and a cantilever-like elastic structure 59 provided with elasticity, at least, at the outer side of its radial direction by means of a U-shaped notch at the front face part of its front portion.

Both surface portions of both cantilever-like elastic structures 58 and 59 are provided with protrusions 58a and 59a whose cross sections viewed in axial directions have triangular tops, to protrude toward the outsides of the radial directions.

At the side of the central part of the back portion of the inner cylinder member 57, a flange part 60 is formed in which a plurality of ring-shaped convex portions, with diameters that stepwise increase compared to the central part, are provided on its outer periphery. When the front portion of the inner cylinder member 57 is installed in the body 2, the front end face of this flange part 60 contacts the back end face of the body 2 in order not to enter the body 2 further, and when the outer cylinder cap 56 is attached to the outside of the back portion of the inner cylinder member 57, the step portion of the inside periphery of the outer cylinder cap 56 is rotatably fitted to the outer periphery of the flange part 60 to prevent its detachment.

Furthermore, the back portion of the inside peripheral face of the outer cylinder cap 56 is formed with a groove in its axial directions and a protrusion 58a on the surface of the cantilever-like elastic structure 58 is fitted to that groove so that the inner-cylinder member 57 and the outer cylinder cap 56 are allowed to rotate integrally at the time of operation and to provide a clutch structure such that the protrusion 58a of the elastic structure 58 is detached from the groove of the outer cylinder cap 56 and relatively rotates with respect to the outer cylinder cap when a critical or greater rotary force is applied.

An engaging part 39 provided on the front portion of the inner cylinder member 57 is a hole having an anisotropic cam shape in which the inside of the central hole of its front portion corresponds to the shape of the outer periphery of the screw stock 32. The screw stock 32 is inserted into and penetrates the central hole of the engaging part 39, whereby the screw stock 32 engages slidably in its axial direction and non-rotatably with respect to the operation conversion part 55 (via the engaging part 39).

Furthermore, the outer peripheral face of the screw stock 32 except a notch portion, which screw stock is inserted into, penetrating, and engaging this engaging part 39, is formed with a male screw, wherein the male screw is engaged with a female screw formed inside the central hole of the retention member 34.

In the present embodiment, the male screw and the female screw are right-hand screws, and when the outer cylinder cap 56 of the operation conversion part 55 is clockwise-rotated with respect to a body 4, the screw stock 32 is clockwise-rotated via the engaging part 39, whereby the male screw of the screw stock 32 moves forward by the female screw of the retention member 34 so that the piston body 35 is pushed and a viscous cosmetic material in the viscosity cosmetic material containment space (storage tank) 2b is pressurized (liquid pressurizing mechanism). On the other hand, when the outer cylinder cap 56 is counter-clockwise-rotated with respect to the body 4, the screw stock 32 is counter-clockwise-rotated via the engaging part 39, whereby the male screw of the screw stock 32 moves backward by the female screw of the retention member 34 so that the piston 35 is pulled and the viscous cosmetic material in the viscous cosmetic material containment space 2b is depressurized (liquid depressurizing mechanism).

Additionally, if necessary, each engaging portion of the screw stock 32 and retention member 34 can be left-hand screws. In that case, the viscous cosmetic material 4 is pressurized by means of counter-clockwise rotation while the viscous cosmetic material 4 is depressurized by means of clockwise rotation, contrary to the above descriptions.

This retention member 34 is a ring-shaped member having a generally double cylinder shape in which a smaller diameter part 34b is fixed on the inside and the tip of a larger diameter part 34a and the outer peripheral face of the larger diameter part 34a is engaged with the inside of the body 2 and attached without rotation. Then, the inner peripheral face of the smaller diameter part 34b of the retention member 34 is formed with a female screw that is a right-hand screw and the inner peripheral face of the larger diameter part 34a is provided such that a fitting part 61 composed of a tooth-profile ratchet groove to which a protrusion 58a of the elastic structure 58 is fitted is formed all over the inner peripheral face.

As shown in FIG. 7(c), the corner portions of the teeth of the fitting part 61 at the side of the inner peripheral portion are smooth on one side and are sharp on the other side. A protrusion 59 of the elastic structure 59 is fitted to the fitting part 61 on the condition that the inner cylinder member 57 is fitted to the retention member 34 from behind.

When the operation conversion part 55 is rotated clockwise (rotated in one direction) (in the direction of arrow R in FIG. 7) with respect to the body 2, the inner cylinder member 57 is clockwise rotated via the outer cylinder cap 56 and the screw stock 32 is clockwise rotated via the engaging part 39 whereby the screw stock 32 moves forward by its engagement with the female screw of the retention member 34 and the piston body 35 is pushed out to provide a viscous cosmetic material in the body in a pressurized state.

The operation conversion part 55, the screw stock 32 and the retention member 34 configure a pressurizing structure rotating lightly while providing a feeling of a click, because the protrusion 59 having been in the state of fitting to the groove between the adjacent teeth contacts the smooth corner portion, passes through that corner portion, and fits to the adjacent tooth, when the inner cylinder member 57 of the operation conversion part 55 is rotated in one direction (rotated in the direction of R). On the other hand, the operation conversion part 55, the screw stock 32 and the retention member 34 configure a depressurizing structure rotating heavily while providing feeling of regulation, because the protrusion 59a having been in the state of fitting to the groove between the adjacent teeth contacts the sharp corner portion, passes through that corner portion, and fits to the adjacent groove when that inner cylinder member 57 is rotated in the other direction (rotated in the anti-R direction) and therefore rotation is not performed unless a rotation force (torque) stronger than that of the one direction is applied.

Thus, both the retention member 34 and the operation conversion part 55 (the forward outer peripheral face of the inner cylinder member 57) in the fitting part 61 are ratcheted to one side and easily rotated to facilitate ejection of the viscous cosmetic material 4 when the ejection is desired. On the other hand, when the viscous cosmetic material 4 is withdrawn into the container after use, rotation in the other direction is allowed.

At the time of depressurization, a torque limiter function is provided to interrupt torque transmission and to enable to a rotation idle state by means of eliminating engagement of the inner peripheral face of the outer cylinder cap 56, the inner cylinder member 57, and the protrusion 58a of the elastic structure 58 in order not to suction the viscous cosmetic material 4 rapidly when a critical or greater rotary force is applied to the operation conversion part 55.

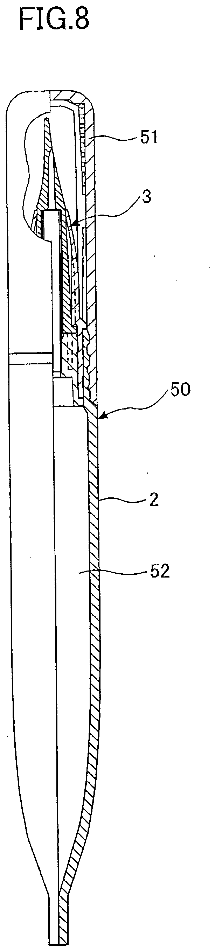

FIG. 8 is a diagram showing a liquid pressurizing mechanism (liquid pressurizing means) for a viscous cosmetic item according to yet another embodiment of the present invention.

A viscous cosmetic item 50 shown in FIG. 8 generally has a similar structure to that of the viscous cosmetic item 1 of FIG. 2, wherein identical reference numerals will be provided to similar parts or analogous parts and their detailed descriptions will be omitted.

As shown in FIG. 8, a cap 51 of the viscous cosmetic item 50 is configured as a double-cap to protect the structure of the leading axle 3 air-tightly.

In the liquid pressurizing mechanism shown in FIG. 8, the body 2 is a flexible viscous cosmetic material storage tank (viscous cosmetic material containment space) 52. In such a manually-operated liquid pressurizing mechanism, the body 2 is pressurized lightly by a finger or the like, so that the viscous cosmetic material 4 is pressurized in the body 2 and the viscous cosmetic material 4 is ejected from a communication path 24 to a planar part (temporary liquid retaining part) 25. Furthermore, even if the body is pressurized somewhat excessively, liquid is retained temporarily to avoid its dripping or the like because the planar part 25 is present. Moreover, a liquid depressurizing mechanism for depressurizing a viscous cosmetic material by eliminating pressurization after the pressurization is configured to enable the withdrawal of an extra viscous cosmetic material on an application part from the ejection port of an application face into the inside of a containment part.

Next, a viscous cosmetic material 4 contained in a container of a viscous cosmetic item according to the present invention will be described in regard to the viscous cosmetic item as described above. In the present example, the viscous cosmetic material 4 is a viscous lip rouge. In the present example, although the amounts of components of the viscous cosmetic material 4 may be changed depending on its formulation examples, a deodorant polybutene, a vaseline, a liquid paraffin, triisostearin, diisostearyl malate, a castor oil, and another liquid oil may be used for oil components and Red No. 202 and safflower red may be used for coloring materials. Additionally, because these are all publicly-known, their detailed descriptions will not be provided hereinafter. Furthermore, the viscous cosmetic materials 4 of the present example are manufactured using a conventionally and publicly known manufacturing method.

In general, if the viscosity of a viscous cosmetic material in a container is too high in a viscous cosmetic item which contains a content in a push-out container capable of withdrawing it, ejection from an ejection port is difficult while the application resistance is so high that it is difficult to spread the cosmetic material on a part to which the cosmetic material is applied, and a feeling of moistness and the like in use is low. On the contrary, if the viscosity of a viscous cosmetic material is too low, it becomes easy to cause bleeding on the part to which the cosmetic material is applied or to cause leakage (liquid dripping) of a viscous cosmetic material from an ejection port. Furthermore, when outside air temperature rises at the time of storage, the viscosity of a viscous cosmetic material is so low that it is easy to cause liquid dripping.

Moreover, the behavior of a viscous cosmetic material may be influenced depending on the viscosity of the viscous cosmetic material and the shape of a container for containing it, so that the problems described above may occur.

Therefore, it is necessary not only to adjust the viscosity of a viscous cosmetic material but also to consider the shape of a container for containing it in order that a viscous cosmetic material moves in a container of a viscous cosmetic item efficiently and the viscous cosmetic material is retained in the container effectively, and therefore, ejection tests were conducted as shown in Table 1 described below which use viscous cosmetic materials with different viscosities and containers with difference pipe diameters of application liquid supply pipes in the container of the viscous cosmetic item. The viscous cosmetic materials were prepared by using an oil component such that their viscosities at 25 °C were between about 50,000 mPa·s and about 100,000 mPa·s.

Furthermore, a container as shown in FIG. 5 was used for a container of viscous cosmetic item which contains the prepared viscous cosmetic material and four kinds of containers were used in which the pipe diameters of application liquid supply pipes were 1.2 mm, 1.7 mm, 2.0 mm, and 2.2 mm.

Table 1 shows the results of ejection tests performed for viscous cosmetic items which contained the above-mentioned viscous cosmetic materials with two kinds of viscosities in application liquid containment spaces 2b of containers of FIG. 5 which have the four kinds of pipe diameters. Additionally, the viscosities of the viscous cosmetic materials were measured by an Ubbelohde-type viscometer (dilution-type glass tube-type viscometer) in atmosphere at 25 °C. Furthermore, it was conducted at a shear rate of 20 sec⁻¹ at a time when the viscous cosmetic materials were ejected from the viscous cosmetic items. This rate corresponds to a shear rate in the case where rotators of the viscous cosmetic items are rotated rapidly.

**[Table 1]**

| Pipe inner diameter (mm) Viscosity at 25 °C (mPa·s) *1 | 1.2 | 1.7 | 2 | 2.2 |
|---|---|---|---|---|
| About 50000 | × | ○ | ○ | ○ |
| About 100000 | × | Δ | ○ | ○ |

| | | | | |
|---|---|---|---|---|
| *1 Ubbelohde viscometer, 25 °C ○ : All amount ejection is possible Δ : Half amount ejection is possible × : Ejection is impossible | | | | |

From Table 1, it was found that the higher the viscosity of a viscous cosmetic material is, the more difficult ejection is, and it is necessary to provide an application liquid supply pipe with a large pipe diameter in a container of a viscous cosmetic item. Thereby, it can be seen that, for example, an application liquid supply pipe with a large pipe diameter of about 5 mm may also be used effectively.

In the present example, a container with an application liquid supply pipe having a pipe diameter of 2.2 mm was fixed and viscous cosmetic material were investigated which is suitable to that pipe diameter in order to move in the container efficiently and to be retained in the container effectively.

To this end, the shear rates and viscosities were measured when ejection was conducted from viscous cosmetic items which contained five kinds of viscous cosmetic material with different viscosities in application liquid containment spaces 2b of containers having a pipe diameter of 2.2 mm in FIG. 5, and a graph shown in FIG. 9 was obtained. As described below, curves with rhombuses, squares, triangles, and circles shown in FIG. 9 correspond to formulation examples 3-1, -2, -3, and -4 shown in Table 3, respectively. Additionally, a curve with * marks shown in FIG. 9 is a comparative example for a viscous cosmetic material which contained a setting agent, but did not contain a thickening agent. Therefore, the viscous cosmetic materials represented by the curves with rhombuses, squares, triangles, and circles in FIG. 9 were viscous cosmetic materials having compositions in Table 3 which were prepared and manufactured in accordance with the respective formulation examples. Additionally, the viscous cosmetic material represented by the curve with * marks in FIG. 9 might have a composition which contained a setting agent but did not contain a thickening agent in one of formulation examples 3-1, -2, -3 and -4 shown in Table 3. Because other components of a viscous cosmetic material, such as an oil component and a coloring material, are publicly-known materials necessary for a cosmetic material as described above, the viscous cosmetic materials of the present examples were manufactured while a conventionally and publicly-known manufacturing method were used.

As described in detail, the viscous cosmetic materials of formulation examples 3-2 and 3-4 in Table 3 are viscous cosmetic material suitable to a container having the above-mentioned shape for the present invention and the viscous cosmetic materials are preferable which have the relationship between a viscosity and a shear rate as indicated by the curve with squares or the curve with circles in FIG. 9.

Meanwhile, although a viscous cosmetic material which has been once ejected onto an application face can be re-contained in a container of a viscous cosmetic item according to the present invention, the antiseptic effect of the viscous cosmetic materials was investigated for preventing secondary contamination caused by microorganisms in the viscous cosmetic materials.

While an antiseptic agent for a viscous cosmetic material can be selected from a dihydric alcohol group such as dipropylene glycol, 1,3-butanediol, isopropylene glycol, propylene glycol, pentanediol or hexylene glycol and compounded, depending on a formulation system of a viscous cosmetic material, the evaluations of the antiseptic effect of the used viscous cosmetic materials 4 which were formulated in accordance with the following composition examples were conducted in the present example and their results are shown in Table 2 described below,

Formulation example 1 was a viscous cosmetic material with a low viscosity and formulation example 2 was a viscous cosmetic material with a high viscosity. The compounding ratios of oil components and coloring materials added to the viscous cosmetic materials 4 were drastically changed in order to provide a difference between the viscosities in formulation examples 1 and 2 but the compounding ratio of a dihydric alcohol as an antiseptic agent was not changed between composition examples 1 and 2, wherein dipropyleneglycol was selected for the dihydric alcohol and their compounding quantities were not changed in the present example wherein 0 wt%, 0.05 wt%, 0.1 wt%, 0.2 wt%, or 0.3 wt% thereof were compounded into both formulation examples 1 and 2.

The viscous cosmetic materials having the formulations in Table 2 were prepared and manufactured in accordance with the respective formulation examples. Additionally, the viscous cosmetic materials of the present examples were manufactured by using a conventionally-known and publicly-known method for manufacturing a viscous cosmetic material, because a viscous cosmetic material was publicly known which contains a dihydric alcohol such as dipropylene glycol as an antiseptic agent thereby preventing secondary contamination caused by microorganisms, and besides, a setting agent, a thickening agent, oil components such as a deodorant polybutene, vaseline, a liquid paraffin, triisostearin, diisostearyl malate, castor oil and other liquid oil components, and coloring materials such as Red No. 202 and safflower red were all publicly-known materials necessary for a cosmetic material. Then, viscous cosmetic items were provided by containing the manufactured 10 examples of viscous cosmetic materials 4, i.e., formulation examples 1-1 to 1-5 and 2-1 to 2-5, in the application liquid containment spaces 2b of the containers as shown in FIG. 5 and evaluations against bacteria, yeast, and fungi were conducted to obtain the results of Table 2 for the respective formulation examples.

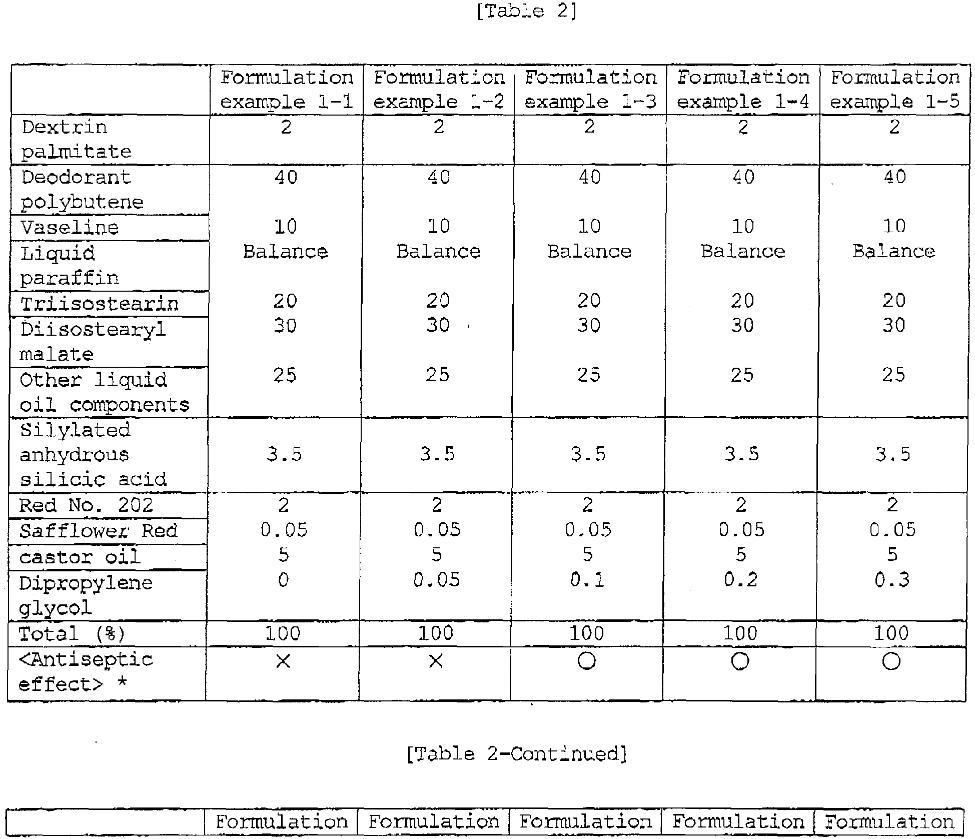

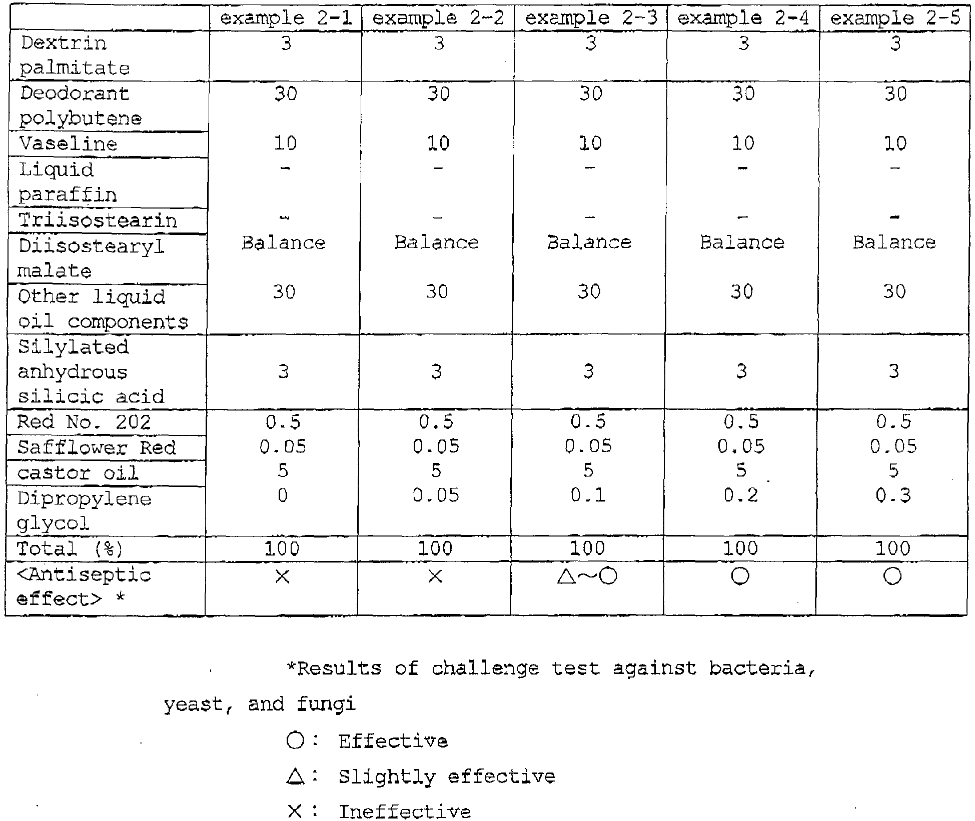

As can be seen from Table 2, it was confirmed that the compounding quantity of dipropylene glycol that is a dihydric alcohol as an antiseptic agent is preferably 0.2 wt% or more to provide sufficient antiseptic effect regardless of whether the viscosity is high or low.

Additionally, it has been known that dihydric alcohols such as 1,3-butanediol, isopropylene glycol, propylene glycol, pentanediol, and hexylene glycol, as well as dipropylene glycol may have a similar antiseptic effect, in some formulation system of a viscous cosmetic material for a viscous cosmetic item of the present invention. Therefore, an antiseptic agent used for a viscous cosmetic material for a viscous cosmetic item of the present invention is not limited to dipropylene glycol but can be selected from a group of these dihydric alcohols and used appropriately, depending on a formulation system of a viscous cosmetic material.

Additionally, ejection tests and antiseptic effect tests were conducted for viscous cosmetic items in which the pipe diameter of an application liquid supply pipe in a container for the present invention as shown in FIG. 5 was 2.2 mm and the viscous cosmetic material 4 formulated as one of compositions 3-1 to 3-4 in Table 3 was contained in the application liquid containment apace 2b, in order to check the viscous cosmetic items of the present invention. Additionally, formulation examples 3-1, 2, 3 and 4 correspond to curves with rhombuses, squares, triangles, and circles shown in FIG. 9, respectively.

**[Table 3]**

| | Formulation example 3-1 | Formulation example 3-2 | Formulation example 3-3 | Formulation example 3-4 |
|---|---|---|---|---|
| Dextrin palmitate | 5 | 2 | 2 | 3 |
| Deodorant polybutene | 40 | 40 | 40 | 40 |
| Vaseline | 10 | 10 | 10 | 10 |
| Liquid paraffin | Balance | Balance | Balance | Balance |
| Triisostearin | 20 | 20 | 20 | 20 |
| Diisostearyl malate | 30 | 30 | 30 | 30 |
| Other liquid oil components | 25 | 25 | 25 | 25 |
| Silylated anhydrous silicic acid | 0 | 3 | 3.5 | 2.5 |
| Red No. 202 | 2 | 2 | 2 | 2 |
| Safflower Red | 0.05 | 0.05 | 0.05 | 0.05 |
| castor oil | 5 | 5 | 5 | 5 |
| Dipropylene glycol | 0.2 | 0.2 | 0.2 | 0.2 |
| Total (%) | 100 | 100 | 100 | 100 |
| Viscosity 1 at 25 °C *1 | 17500 | 20700 | 25400 | 19000 |
| Viscosity 2 at 25 °C *2 | 13300 | 10420 | 11920 | 10280 |
| Ejection Test *3 | × | ○ | Δ | ○ |
| <Antiseptic effect> *4 | ○ | ○ | ○ | ○ |

| | | | | |
|---|---|---|---|---|
| *1 Values at shear rate of 5 sec⁻¹ (mPa·s) (E-type viscometer) *2 Values at shear rate of 40 sec⁻¹ (mPa·s) (E-type viscometer) *3 Ejection conditions at a pipe inner diameter of 2.0 mm and at 0 °C ○ : Ejection is good Δ : Ejection is defective × : Ejection is impossible *4 Results of challenge test against bacteria, yeast, and fungi ○ : Effective Δ : Slightly effective × : Ineffective | | | | |

It was found that the viscous cosmetic materials 4 manufactured in accordance with the formulations in Table 3 had high viscosities in the case where the shear rate is low, and was a viscous cosmetic material suitable to a container of a viscous cosmetic item of the present invention in which the pipe diameter of an application liquid supply pipe was 2.2 mm, by means of the formulation of a composition with a low viscosity in the case where the shear rate is high. Additionally, although the pipe diameter of an application liquid supply pipe of a container was 2.2 mm in the present examples, an application liquid supply pipe with a large pipe diameter of, for example, about 5 mm can also be used for a container.

Therefore, the viscous cosmetic materials of formulations 3-2 and 3-4 are viscous cosmetic materials suitable to a container for the present invention which had the above-mentioned shape, and the viscous cosmetic materials are preferable which have the relationships between the viscosity and the shear rate as indicated by the curve with squares and the curve with circles shown in FIG. 9. That is, it is found that a viscous cosmetic material suitable to the shape of a container for the present invention is provided by having a viscosity of 19000 mPa·s or greater for a viscous cosmetic material at rest, that is, at a shear rate of 0 to 5 sec⁻¹ and having a viscosity of 11000 mPa·s or less at a high shear rate, that is, at a shear rate of 40 sec⁻¹, which is a common shear rate that is practically used by a user. In particular, as can also be seen in FIG. 3, 2 wt% to 3 wt% of dextrin palmitate as a setting agent is compounded and 2.5 wt% to 3.0 wt% of silylated anhydrous silicic acid as a thickening agent is compounded, so that the viscous cosmetic material has a viscosity in these ranges.

Furthermore, it was confirmed that the viscous cosmetic materials 4 of formulation examples 3-2 and 3-4 having the viscosities in the above-mentioned ranges and further having 0.2 wt% of dipropylene glycol compounded also had sufficient antiseptic effect for preventing secondary contamination caused by microorganisms.

Although the viscous cosmetic material for the viscous cosmetic item of the present invention was a viscous lip rouge in the present example, it is not limited to this material but is allowed to be of application on a part of the skin, for example, a concealer or the like, and can have any configuration without departing from the scope of the present invention.

Thus, there is provided a viscous cosmetic item with an internal block installed in a containment part which is allowed to push a viscous cosmetic material outward from an ejection port provided on an application face of an application part by providing the tip of a container with the application part and rotating a rotator located at a part of the container in one direction thereby pressurizing the viscous cosmetic material contained in the containment part and is allowed to withdraw a viscous cosmetic material remaining on the application part from the ejection port of the application face to the containment part by rotating the rotator in the reverse direction, wherein the container is characterized in that it is a push-out container capable of withdrawing a content, including the application part being composed of an elastic material, the application face being an inclined plane face inclined with respect to the longitudinal axis of the container body, the diameter of a cylinder viscous cosmetic material supply pipe of the container being 1.7 to 2.2 mm, and the ejection port communicated therewith and being for ejecting the viscous cosmetic material outward being provided on the application face at an open state. Furthermore, a viscous cosmetic material contained in a container is also characterized in that it has a viscosity of 19000 mPa·s or greater for a viscous cosmetic item at rest, that is, at a shear rate of 0 to 5 sec⁻¹ and has a viscosity of 11000 mPa·s or less at a high shear rate, that is, at a shear rate of 40 sec⁻¹ that is a common shear rate used by a user practically, and which is prepared in a formulation including a dihydric alcohol, such as dipropylene glycol, with a compounding quantity of 0.2 wt% to prevent secondary contamination caused by microorganisms. Therefore, only a suitable amount of a viscous cosmetic material for makeup can be used without waste in a viscous cosmetic item of the present invention, because the viscous cosmetic material in the container can move efficiently and the viscous cosmetic material is retained in the container effectively while a rotator provided on a part of the container including a containment part is rotated in one direction or the reverse direction thereto such that an internal block arranged in the containment part is moved up and down.

Moreover, there is provided a viscous cosmetic item in which a viscous cosmetic material prepared with a dihydric alcohol, such as dipropylene glycol, with a compounding quantity of 0.2 wt% or more is contained in a containment part in addition to the above-mentioned moderate viscosities, whereby an application face after use is prevented from being unsanitary, in particular, even if a cosmetic material remaining on an application part after makeup is re-contained in the containment part by rotating a rotator in the reverse direction, and it is possible to provide effective prevention of secondary contamination caused by microorganisms, to have antiseptic effect against contamination and the like at the time of storage, to keep an unused viscous cosmetic material in a good state and to prevent degradation of a product, due to the effect of 0.2 wt% or more of the compounded dihydric alcohol, such as dipropylene glycol.

Furthermore the viscous cosmetic items of the present invention are allowed to be a variety of viscous cosmetic items due to combinations of the above-mentioned containers and viscous cosmetic materials.

The preferred examples of the present invention have been described above in detail but the present invention is not limited to such specific embodiments and various alterations or modifications are allowed within the scope of the present invention as recited in the claims.

## Claims

1. A viscous cosmetic item comprising: a push-out container (2) and a viscous cosmetic material (4) contained in the push-out container, the push-out container (2) forming a cylinder-shaped containment part and being capable of withdrawing a content, the push-out container (2) comprising an application part (10a) provided at a tip of this container, a rotator (31) located on a part of the container, and an internal block (35) installed in the containment part, the rotator (31) being provided such that the rotator located on a part of the container is configured to rotate in one direction thereby pressurizing the viscous cosmetic material (4) contained in the containment part so as to make it possible to push the viscous cosmetic material outward from an ejection port (24a) provided on an application face of the application part (10a) and such that the rotator (31) is configured to rotate in a reverse direction thereby making it possible to withdraw an extra viscous cosmetic material (4) on the application part (10a) from the ejection port (24a) of the application face into the containment part,
wherein the viscous cosmetic item (4) comprises the application part (10a) being composed of an elastic material, the application face being an inclined plane face inclined with respect to a longitudinal axis of a container body (2), a cylinder-shaped viscous cosmetic material supply pipe (13) of the container,
**characterised in that** the cylinder-shaped viscous cosmetic material supply pipe (13) has a diameter of 1.7 to 2.2 mm, the ejection port (24a), for ejecting the viscous cosmetic material (4) outward is communicated therewith and provided on the application face at an open state, and the viscous cosmetic material (4) contained in the containment part of the container (2) has a viscosity of 19000 mPa·s or greater at a shear rate of 0 to 5 sec⁻¹ and a viscosity of 11000 mPa·s or less at a shear rate of 40 sec⁻¹.

2. The viscous cosmetic item as claimed in claim 1, wherein the viscous cosmetic material (4) comprises 0.2 wt% or more of dipropylene glycol.

## Patentansprüche

1. Viskoser Kosmetikartikel, der Folgendes umfasst: einen Ausdrückbehälter (2) und ein in dem Ausdrückbehälter enthaltenen viskoses kosmetisches Material (4), wobei der Ausdrückbehälter (2) einen zylinderförmigen Aufnahmeteil bildet und einen Inhalt zurückziehen kann, wobei der Ausdrückbehälter (2) einen an einer Spitze dieses Behälters vorgesehenen Applikationsteil (10a), einen an einem Teil des Behälters befindlichen Rotator (31) und einen im Aufnahmeteil installierten internen Block (35) umfasst, wobei der Rotator (31) so vorgesehen ist, dass der an einem Teil des Behälters befindliche Rotator zum Drehen in einer Richtung konfiguriert ist, um dadurch das im Aufnahmeteil enthaltene viskose kosmetische Material (4) unter Druck zu setzen, um es zu ermöglichen, das viskose kosmetische Material aus einer Auswurföffnung (24a) auszustoßen, die an einer Applikationsfläche des Applikationsteils (10a) vorgesehen ist, und so, dass der Rotator (31) zum Drehen in einer Umkehrrichtung konfiguriert ist, um es dadurch zu ermöglichen, zusätzliches viskoses kosmetisches Material (4) am Applikationsteil (10a) von der Auswurföffnung (24a) der Applikationsfläche in den Aufnahmeteil zurückzuziehen,
wobei der viskose Kosmetikartikel (4) den Applikationsteil (10a) bestehend aus einem elastischen Material umfasst, wobei die Applikationsfläche eine geneigte ebene Fläche ist, die mit Bezug auf eine Längsachse eines Behälterkörpers (2) geneigt ist, eine zylinderförmige Hülse (13) des Behälters zum Zuführen von viskosem kosmetischem Material,
**dadurch gekennzeichnet, dass** die zylinderförmige Hülse (13) zum Zuführen von viskosem kosmetischem Material einen Durchmesser von 1,7 bis 2,2 mm hat, wobei die Auswurföffnung (24a) zum Auswerfen des viskosen kosmetischen Materials (4) nach außen damit in Verbindung ist und auf der Applikationsfläche in einem offenen Zustand vorliegt, und das im Aufnahmeteil des Behälters (2) enthaltene viskose kosmetische Material (4) eine Viskosität von 19.000 mPa.s oder höher bei einer Scherrate von 0 bis 5 sec⁻¹ und eine Viskosität von 11.000 mPa.s oder weniger bei einer Scherrate von 40 sec⁻¹ hat.

2. Viskoser Kosmetikartikel nach Anspruch 1, wobei das viskose kosmetische Material (4) 0,2 Gew.-% oder mehr Dipropylenglykol umfasst.

## Revendications

1. Article cosmétique visqueux comprenant : un réservoir à sortie par poussée (2) et un matériau cosmétique visqueux (4) contenu dans le réservoir à sortie par poussée, le réservoir à sortie par poussée (2) formant une partie de rétention de forme cylindrique et permettant de retirer un contenu, le réservoir à sortie par poussée (2) comprenant une partie applicatrice (10a) prévue à une extrémité dudit réservoir ; un organe rotatif (31) situé sur une partie du réservoir ; et un bloc intérieur (35) installé dans la partie de rétention, l'organe rotatif (31) étant prévu tel que l'organe rotatif situé sur une partie du réservoir est configuré pour tourner dans une direction et exercer ainsi une pression sur le matériau cosmétique visqueux (4) contenu dans la partie de rétention de façon à pouvoir pousser le matériau cosmétique visqueux vers l'extérieur à partir d'un orifice d'éjection (24a) prévu sur une face d'application de la partie applicatrice (10a), et tel que l'organe rotatif (31) est configuré pour tourner dans une direction opposée de façon à pouvoir retirer un reste de matériau cosmétique visqueux (4) sur la partie applicatrice (10a) depuis l'orifice d'éjection (24a) de la face d'application dans la partie de rétention,
dans lequel l'article cosmétique visqueux (4) comprend la partie applicatrice (10a), laquelle est composée d'un matériau élastique, la face d'application étant une face plane oblique inclinée par rapport à un axe longitudinal d'un corps de réservoir (2), un tube d'alimentation de matériau cosmétique visqueux (13) de forme cylindrique du réservoir,
**caractérisé en ce que** le tube d'alimentation de matériau cosmétique visqueux (13) de forme cylindrique a un diamètre de 1,7 à 2,2 mm, **en ce que** l'orifice d'éjection (24a) est mis en communication avec lui pour éjecter le matériau cosmétique visqueux (4) vers l'extérieur et est prévu sur la face d'application dans un état ouvert, et **en ce que** le matériau cosmétique visqueux (4) contenu dans la partie de rétention du réservoir (2) a une viscosité égale ou supérieure à 19 000 mPa·s à une vitesse de cisaillement de 0 à 5 s⁻¹ et une viscosité inférieure ou égale à 11 000 mPa·s à une vitesse de cisaillement de 40 s⁻¹.

2. Article cosmétique visqueux selon la revendication 1, dans lequel le matériau cosmétique visqueux (4) comprend 0,2 % en poids ou plus de dipropylène glycol.
